# DEMANDE DE BREVET EUROPEEN

(11) **EP 0 527 282 A1**
(43) Date de publication de la demande: **17.02.1993**
(21) Numéro de dépôt: 91440066.8
(22) Date de dépôt: 13.08.1991
(51) Int. Cl.: B65F 3/00, B65F 1/00, B65F 9/00, B65F 7/00, A61B 19/02

(54) **Collecte des déchets hospitaliers**

(71) Demandeur: NICOLETTI AGREGATS S.A., F-06100 Nice (FR); SOGEDET SaRL, F-78000 Versailles (FR)
(72) Inventeur: Nicoletti, Didier, F-06200 Nice (FR); Donche, Jean-Pierre, F-06000 Nice (FR)
(74) Mandataire: Hammond, William

(57) **Abrégé**

Procédé pour la collecte sélective et contrôlée des déchets hospitaliers de toute origine sur un territoire donné, en vue de leur traitement et/ou destruction, généralement par incinération, caractérisé en ce qu'il consiste :
- dans un premier temps, à recueillir les déchets à leur point d'origine dans un volume jetable parfaitement étanche et non perforable,
- dans un second temps, à placer ce volume plein dans un réceptacle fixe verrouillable, équipé de moyens de stérilisation,
- dans un troisième temps, à collecter les volumes jetables présents dans l'ensemble des réceptacles fixes répartis sur ledit territoire,
- dans un quatrième temps, à éditer et placer sur chaque volume jetable collecté une étiquette identifiant l'origine et la nature dudit volume, à partir d'un lecteur de code-barres incluant une horloge et une unité d'impression de telles étiquettes,
- dans un cinquième temps, l'ensemble des volumes jetables ainsi collectés et identifiés sont regroupés par groupes de nombres croissants, en vue de leur transport jusqu'au site de destruction où, après contrôle, ils sont incinérés.

## Description

La présente invention concerne un système destiné à permettre la collecte sélective et contrôlée des déchets hospitaliers de toute origine en vue de leur destruction.

Par "déchets hospitaliers", on désigne globalement tous les produits résiduels de l'activité médicale, qu'il s'agisse des produits constamment rejetés par les établissements hospitaliers et assimilés ou des produits du même genre rejetés occasionnellement par les médecins et/ou les professionnels de la santé, ainsi que par les particuliers, tels que ouates ou gazes souillées, restes de médicaments ou de vaccins, seringues polluées et produits analogues dits "à risques" qui, pour des raisons évidentes constituent des sources de contamination et même des dangers pour la santé publique s'ils tombent entre des mains non averties.

Actuellement, seuls certains établissements hospitaliers et assimilés détruisent directement ces produits dans des fours d'incinération sur place sans pour autant contrôler d'une manière systématique la chaîne de collecte interne de déchets, ou, pour quelques autres, par l'intermédiaire d'un système de collecte qui conduisent ces produits vers des unités spécifiques de traitement. Mais pour la plupart des établissements hospitaliers et assimilés, la quasi-totalité des cabinets de soins, des organismes de soins à domicile, et de manière générale, pour toutes les professions libérales de santé ainsi que pour les particuliers, les déchets de type hospitalier suivent le circuit des ordures ménagères (c.à.d. sacs poubelles, poubelles, conteneurs placés sur voie publique pour être collectés par des bennes à ordures et ensuite être incinérés ou mis en décharge, dans la plupart des cas) et ceci sans contrôle particulier avec tous les risques que cela comporte.

Il en résulte un danger permanent, que l'invention permet d'écarter par un ensemble de moyens par lesquels l'intégralité des déchets hospitaliers produits sur un territoire déterminé sont systématiquement collectés à leur point d'origine et amenés à un point de traitement et/ou de destruction généralement par incinération, sous un contrôle permanent permettant à la fois de maintenir une hygiène absolue et de détecter à tout moment tout incident ou toute anomalie résultant d'une irrégularité dans le processus, par exemple le détournement de certains de ces produits en vue de leur réutilisation, tels que les seringues, ou la perte de produits dangereux, tels que les résidus de vaccins ou de médicaments.

Cet ensemble de moyens correspondent au déroulement d'un processus garantissant cette sécurité et ce contrôle, et qui va faire l'objet de la Description ci-après :
- Dans un premier temps, au point d'origine des déchets, c'est-à-dire soit un service hospitalier, notamment chirurgical, soit un cabinet médical ou dentaire, et d'une manière générale tout professionnel de santé en contact avec des patients, ces déchets sont placés dans un volume jetable à usage unique présentant la solidité et l'étanchéité suffisantes pour accepter à la fois les résidus médicamenteux, les pansements contaminés, les seringues usagées et analogues, sans aucune possibilité ou risque d'accès à ces produits une fois ledit volume fermé. Un tel volume peut être réalisé en carton ou matière plastique ; il en existe plusieurs types sur le marché tel celui décrit au brevet italien 20447A87, et qui consiste en un fond replié, et un couvercle également replié, réunis par un manchon latéral. Le plus souvent, le volume lui-même reçoit un sac plastique contenant les déchets. Selon une variante applicable à un traitement semblable de déchets occasionnels, provenant par exemple de patients individuels, un tel volume pourra être le conditionnement même dans lequel un produit est délivré par le pharmacien.
- Dans un second temps, le volume jetable est placé dans une borne réceptacle fixe, où le personnel de collecte va le récupérer.
cette borne réceptacle du jetable est rattachée à un producteur bien défini de déchets de type hospitalier. Ce producteur peut être un professionnel de la santé du genre médecin, dentiste, infirmière libérale, vétérinaire, etc... ou un service particulier dans un établissement hospitalier et assimilé.

Ses caractéristiques fondamentales sont les suivantes :

### - Identification de la borne

La borne réceptacle du jetable est munie d'une adresse en clair portant un ensemble de données telles que : activité médicale, lieu de celle-ci, référence du producteur, etc..., de manière à pouvoir enregistrer cette adresse au moment de la collecte, l'ensemble de ces renseignements étant traduits en code-barres placés en un endroit visible et accessible de la borne.

### - Aspect

La borne est réalisée de préférence en matière plastique moulée ou similaire avec un minimum d'aspérités et sans aucun recoin. Son ouverture est facile. Sa contenance est de plusieurs types de manière à être adaptée aux différents producteurs de déchets.

### - Implantation

La borne est placée dans un lieu facilement accessible au personnel de collecte:
. Pour les professionnels de la santé tels que cabinets de soins, professions libérales, et.... : en ce qui concerne les habitations collectives, la borne est placée dans le local poubelles, par exemple où elle est scellée, par contre, pour les habitations individuelles, elles est placée à la limite du domaine public/privé.
. Pour les services hospitaliers et assimilés, la borne est placée dans un endroit légèrement en retrait de la circulation.

### - Protection

Le contenu de la borne ne doit être accessible que par le producteur et le collecteur, et eux seulement. Pour répondre à cet impératif, et, par ailleurs, pour indiquer si la borne est supposée contenir des déchets hospitaliers, ou ne pas en contenir du tout, une serrure de sécurité, spécialement conçue à cet effet, protège la porte/couvercle.

Dans son principe, cette serrure de sécurité est du type à double clé, à savoir :
· Un système à double clé, comprenant une première clé au moyen de laquelle, après introduction du volume jetable plein, le réceptacle fixe est verrouillé, pour empêcher toute reprise accidentelle ou malveillante. Cet état "verrouillé, c'est-à-dire "plein" du réceptacle, est indiqué par un voyant , par exemple une lumière rouge. Seule une seconde clé, détenue par le personnel de collecte, permet d'ouvrir le réceptacle et d'en extraire le volume jetable. La manoeuvre de cette seconde clé est signalée par un autre voyant, par exemple une lumière verte, qui indique que le réceptacle est à nouveau disponible.

Selon un exemple non limitatif de réalisation pratique, la serrure fonctionne de la manière suivante :
Le producteur possède une clé à gauche fermant dans le sens contraire des aiguilles d'une montre.

Le collecteur possède un clé à droite fermant dans le sens des aiguilles d'une montre.

Par ce système et par des lumières judicieusement disposées et un mécanisme particulier (languette portant des disques de couleurs associée à la rotation de la clé provoquant la fermeture), en fermant, le producteur fait apparaître un disque rouge, tandis que le collecteur fait apparaître, quant à lui, un disque vert.

Par rapport au système dit des "toilettes" auquel ce système pourrait être comparé, le code de couleurs vert/rouge indique non pas que la boîte est ouverte ou fermée, mais par qui elle a été fermée (possesseur de clé à gauche ou possesseur de clé à droite).

Pour les bornes placées dans des services hospitaliers, par exemple, et de ce fait nécessitant un grand nombre d'ouvertures et de fermetures, le système mécanique envisagé ci-dessus peut être complété par des clés à signature magnétique ou électronique permettant également de faire apparaître des informations complémentaires aux utilisateurs de la borne.

### - Désinfection

Enfin, la borne est équipée d'un dispositif automatique de désinfection systématique à chaque utilisation. Par exemple, une bombe aérosol (placée dans un emplacement moulé dans la borne) pulvérise le produit désinfectant grâce à son bouton poussoir actionné automatiquement à chaque ouverture ou fermeture de la porte couvercle par un système de came placée sur la porte couvercle.
- Dans un troisième temps, le personnel de collecte, ayant ouvert le réceptacle et prélevé le volume jetable rempli de déchets, procède à l'édition d'une étiquette d'identification et de contrôle de ce volume.

A cet effet, le réceptacle porte une adresse en code-barres relative au producteur de déchets qui lui ont été rattaché (identité, nature du service, etc..., soit une manière générale, toutes les informations relatives à l'origine et la nature des déchets de type hospitalier déposés dans la borne réceptacle par l'intermédiaire du jetable).

Cette adresse est lue grâce à un lecteur spécial qui édite une étiquette en code-barre autocollante reprenant les informations de l'adresse ainsi que la date et l'heure de l'opération de collecte (horloge incorporée au lecteur), puis cette étiquette est collée sur le jetable ainsi collecté. Ce lecteur comporte également un système d'enregistrement de manière qu'à l'issue du transport du volume jetable, l'ensemble des informations enregistrées permet le contrôle des opérations effectuées pendant une période donnée sur un territoire donné à partir d'origines données, l'étiquette autocollante placée sur le volume jetable étant lue avant que ce dernier ne soit traité. Ce lecteur est nouveau et original en lui-même et fait l'objet d'une Demande de brevet séparée de la Déposante.

Dans un 4e temps, et ceci essentiellement pour les volumes jetables provenant des bornes réceptacles installées auprès des producteurs tels que cabinets de soins, organismes de soins à domicile et, d'une manière générale, des professions libérales de santé, ces dits volumes jetables sont regroupés en vue de leur transport jusqu'au site de destruction et ceci afin de réduire le temps de collecte
A cet effet, il est prévu de procéder à de tels regroupements par quantités croissantes, par exemple d'abord à l'aide de "tiroirs" pouvant contenir 2 volumes jetables, chaque "tiroir" étant par lui-même portable, un collecteur pouvant, sans problème, porter 2 "tiroirs", c'est à dire 4 volumes jetables. Ensuite ces "tiroirs" sont regroupés dans un "casier" pouvant recevoir 4 tiroirs, ce "casier" étant manutentionné facilement par un chariot de manutention de type "diable".

Chaque "casier" trouve à son tour sa place dans un "caisson" pour son transport vers le lieu de traitement des déchets hospitaliers, un tel "caisson" étant placé sur un véhicule léger spécialement aménagé à cet effet. Un "caisson" comporte en général 8 "casiers", chaque "casier" comportant 4 "tiroirs", chaque tiroir pouvant recevoir 2 volumes jetables, ce qui fait, pour "un caisson", un total de 64 volumes jetables.

C'est à l'arrivée sur le site de traitement, en principe incinération, de telles charges de 64 volumes jetables, que se fait la lecture et le contrôle informatique des informations éditées pour chaque volume jetable (étiquette autocollante) par le lecteur précité. Ainsi, sont constatées, par exemple, l'absence d'un volume jetable provenant d'un point d'origine collecté, et autres anomalies.

Pour permettre un contrôle systématique complet de la collecte des déchets hospitaliers, les moyens techniques concernant :
* la lecture de l'adresse,
* le marquage (étiquette autocollante),
* l'enregistrement de données,
* la lecture de données avant incinération et
* le traitement des informations ainsi enregistrées,
tels que définis ci-dessus sont étendus, d'une part, aux cartons de déchets hospitaliers compactés ainsi qu'aux fûts spécifiques de ramassage de déchets hospitaliers ne pouvant pas être compactés, le tout provenant des établissements hospitaliers et assimilés, et, d'autre part aux bornes relais placées dans des endroits judicieux (par exemple les pharmacies) permettant le rassemblement des volumes jetables individuels provenant des particuliers.

## Revendications

1. Procédé pour la collecte sélective et contrôlée des déchets hospitaliers de toute origine sur un territoire donné, en vue de leur traitement et/ou destruction, généralement par incinération, caractérisé en ce qu'il consiste :
- dans un premier temps, à recueillir les déchets à leur point d'origine dans un volume jetable parfaitement étanche et non perforable,
- dans un second temps, à placer ce volume plein dans un réceptacle fixe verrouillable, équipé de moyens de stérilisation,
- dans un troisième temps, à collecter les volumes jetables présents dans l'ensemble des réceptacles fixes répartis sur ledit territoire,
- dans un quatrième temps, à éditer et placer sur chaque volume jetable collecté une étiquette identifiant l'origine et la nature dudit volume, à partir d'un lecteur de code-barres incluant une horloge et une unité d'impression de telles étiquettes,
- dans un cinquième temps, l'ensemble des volumes jetables ainsi collectés et identifiés sont regroupés par groupes de nombres croissants, en vue de leur transport jusqu'au site de destruction où, après contrôle, ils sont incinérés.

2. Matériel d'ensemble pour la mise en oeuvre du procédé selon la revendication 1, caractérisé en ce qu'il comprend :
- des volumes jetables souples ou pliables parfaitement étanches et non perforables,
- des réceptacles fixes destinés à recevoir ces volumes jetables et équipés de moyens de verrouillage de sécurité interdisant leur accès à des personnels non autorisés et des moyens de désinfection / stérilisation automatique intervenant à chaque fermeture du couvercle,
- un appareil lecteur de code-barres comportant une horloge intégrée et un système intégré d'édition d'étiquettes, comportant toutes les informations relatives à l'origine et la nature de chaque volume jetable,
- un matériel de groupage des volumes jetables en vue de leur amenée sur le site de destruction.
